# EUROPEAN PATENT APPLICATION

(11) **EP 4 596 038 A1**
(43) Date of publication of application: **06.08.2025**
(21) Application number: 23872197.1
(22) Date of filing: 22.09.2023
(51) Int. Cl.: A61N 5/10, A61B 90/17

(54) **TONGUE SUCTION RESTRAINING DEVICE, MANUFACTURING KIT OF TONGUE SUCTION RESTRAINING DEVICE, AND MANUFACTURING METHOD OF TONGUE SUCTION RESTRAINING DEVICE**

(30) Priority: 30.09.2022 JP 2022158216
(71) Applicant: INSTITUTE OF SCIENCE TOKYO, Tokyo 152-8550 (JP)
(72) Inventor: NAGANO, Takuya, Tokyo 152-8550 (JP); HARAGUCHI, Mihoko, Tokyo 152-8550 (JP)
(74) Representative: EP&C
(86) International application number: PCT/JP2023/034571
(87) International publication number: WO 2024/070971

(57) **Abstract**

A tongue suction restrainer includes an upper fixing portion that is fixable to an upper jaw of a user when the tongue suction restrainer is attached to an oral cavity, an annular abutment portion that abuts on a hard palate of the user when the tongue suction restrainer is attached to the oral cavity, a recess formed at an inner side of the abutment portion to form a closed space with the hard palate in a state in which an entire circumference of the abutment portion abuts on the hard palate, a suction portion communicating with the recess to apply a negative pressure to the closed space, and a tongue suction portion formed at a position facing a tongue of the user when the tongue suction restrainer is attached to the oral cavity, has a suction hole provided to communicate with the recess, and that sucks the tongue by the negative pressure.

## Description

### Technical Field

The present disclosure relates to a tongue suction restrainer, a tongue suction restrainer manufacturing kit, and a method of manufacturing a tongue suction restrainer.

### Background Art

The specification of CN211796633C discloses a tongue restrainer for radiation therapy. This tongue restrainer has an outer tube and an inner tube, and a bottom plate is welded to a bottom portion of the outer tube. A suction hole for adsorbing a tongue is provided at the bottom portion of the bottom plate. The outer tube and the inner tube move relative to each other horizontally, and thus, the overall length of the tongue restrainer is adjusted in accordance with different patient situations. In addition, the tongue restrainer includes a mouthpiece. The mouthpiece is aligned with a tooth in an oral cavity and stably holds the tongue restrainer in the oral cavity. The tongue is restrained by adsorbing the tongue by a negative pressure acting through an adsorption bore. In addition, WO2009/064914A discloses a tongue pulling device for coping with sleep apnea.

### SUMMARY OF INVENTION

### Technical Problem

In Japan, about 5000 people suffer from a tongue cancer per year. Half of these people are early. There are only a few facilities in Japan that perform brachytherapy for early tongue cancer, and it cannot cope with the needs of early tongue cancer. Thus, at present, surgical resection has become a substantial standard of care. In the case of surgical resection, a 5-year survival rate of early tongue cancer exceeds 80%, but in a case in which a surgical resection range is wide, quality of life (QOL) such as speech disorder, intraoral discomfort, and aesthetic problems may remain as a problem.

On the other hand, stereotactic radiation therapy has been found to be effective for early lung cancer, and indicated diseases have been gradually expanded. However, the stereotactic radiation therapy has not yet been put to practical use in a head and neck cancer area. Early tongue cancer is surrounded by muscles with a high tolerable dose, and is considered to be a good indication for the stereotactic radiation therapy. However, since the tongue moves easily, it has been difficult to properly restrain the tongue. It is considered that there is still room for improvement in the tongue restrainer as in the above-described related example.

An object of the disclosure is to appropriately restrain a tongue in order to improve irradiation accuracy of radiation when stereotactic radiation therapy is performed on the tongue. Solution to Problem

A tongue suction restrainer according to a first aspect includes an upper fixing portion that is fixable to an upper jaw of a user when the tongue suction restrainer is attached to an oral cavity of the user, an annular abutment portion that abuts on a hard palate of the user when the tongue suction restrainer is attached to the oral cavity, a recess that is formed inside at an inner side of the abutment portion to form a closed space with the hard palate in a state in which an entire circumference of the abutment portion abuts on the hard palate, a suction portion that communicates with the recess to apply a negative pressure to the closed space from an outside, and a tongue suction portion that is formed at a position facing a tongue of the user when the tongue suction restrainer is attached to the oral cavity, has a suction hole provided to communicate with the recess, and that sucks the tongue by the negative pressure.

The tongue suction restrainer is fixed to the upper jaw of the user by the upper fixing portion when the tongue suction restrainer is attached to the oral cavity of the user, and the annular abutment portion abuts on the hard palate of the user. In a state in which the entire circumference of the abutment portion abuts on the hard palate, the closed space is formed between the recess at an inner side of the abutment portion and the hard palate. The suction portion communicates with the recess, and when the negative pressure is applied from the suction portion to the closed space, the abutment portion is adsorbed to the hard palate. In addition, since the negative pressure acts on the tongue suction portion through the suction hole, a surface of the tongue is sucked and restrained by the tongue suction portion. In particular, the structure described in WO2009/064914A does not include an aspect of the tongue suction restrainer having the recess that is formed at an inner side of the abutment portion to form the closed space with the hard palate in a state in which the entire circumference of the abutment portion abuts on the hard palate. As a result, since the movement of the tongue can be appropriately suppressed, it becomes easy to perform stereotactic radiation therapy on tongue cancer.

According to a second aspect, in the tongue suction restrainer according to the first aspect, the upper fixing portion is fixable to a tooth of the upper jaw of the user.

In this tongue suction restrainer, the upper fixing portion is fixable to the tooth of the upper jaw of the user. Thus, the tongue suction restrainer can be accurately attached to the oral cavity of the user.

According to a third aspect, the tongue suction restrainer according to the first aspect further includes a lower fixing portion that is fixable to a tooth of a lower jaw of the user.

Since the tongue suction restrainer further includes the lower fixing portion that is fixable to the tooth of the lower jaw of the user, the tongue suction restrainer can be more stably attached to the oral cavity of the user.

According to a fourth aspect, in the tongue suction restrainer according to the third aspect, the tongue suction restrainer further includes a protrusion that protrudes from an inner peripheral portion of the lower fixing portion to form a pocket for allowing a part of the tongue to enter between the protrusion and the tongue suction portion. The suction hole is provided in a portion of the lower fixing portion which forms the pocket.

In this tongue suction restrainer, the pocket is formed between the protrusion provided on an inner peripheral portion of the lower fixing portion and the suction portion. A part of the tongue is inserted, for example, a tumor, is into this pocket, and thus, the tumor can be wrapped with the tongue suction restrainer. As a result, air artifacts on the lower jaw side can be reduced, and the tumor can be easily observed by MRI.

In addition, since the suction hole is also provided in the portion of the lower fixing portion that forms the pocket, the tongue can be sucked even in the pocket, and the adhesion between the tongue and the tongue suction restrainer can be enhanced.

A tongue suction restrainer manufacturing kit according to a fifth aspect includes an upper fixing portion material for forming an upper fixing portion that covers and is fixed to a tooth of an upper jaw of a user from a tooth mold of the upper jaw of the user, and forming an annular abutment portion that abuts on a hard palate of the user and a recess that forms a closed space with the hard palate at an inner side of the abutment portion in a state in which an entire circumference of the abutment portion abuts on the hard palate, a tongue suction portion material that is attached to a back surface side of the recess and that forms a tongue suction portion facing a tongue of the user, and a lower fixing portion material for forming a lower fixing portion that is attached to a lower jaw side of the upper fixing portion and that covers and is fixed to a tooth of a lower jaw of the user. A suction hole communicating from the recess to the tongue suction portion and a suction portion communicating with the recess to apply a negative pressure to a closed space from an outside are formed.

In this tongue suction restrainer manufacturing kit, the upper fixing portion is made of the upper fixing member material, and the tongue suction portion is formed on the back surface side of the recess of the upper fixing portion with the tongue suction member material. Further, the lower fixing portion is manufactured on the lower jaw side of the upper fixing portion with the lower fixing member material. As a result, the tongue suction restrainer corresponding to each user can be easily manufactured.

A method of manufacturing a tongue suction restrainer according to a sixth aspect includes, by using the tongue suction restrainer manufacturing kit according to the fifth aspect, a step of forming, by the upper fixing portion material, an upper fixing portion that covers and is fixed to a tooth of an upper jaw of the user from a tooth mold of the upper jaw of the user, a step of forming, by the tongue suction portion material, a tongue suction portion facing a tongue of the user on a back surface side of the recess in the upper side fixing portion, a step of forming, by the lower fixing portion material, a lower fixing portion that covers and is fixed to a tooth of the lower jaw of the user on a lower jaw side of the upper fixing portion, and a step of forming a suction hole communicating with the tongue suction portion from the recess, and a suction portion communicating with the recess and applying a negative pressure to the closed space from an outside.

In the method of manufacturing a tongue suction restrainer, the tongue suction restrainer corresponding to each user can be easily manufactured by using the tongue suction restrainer manufacturing kit.

### Advantageous Effects of Invention

According to the disclosure, it is possible to improve the irradiation accuracy of radiation when the stereotactic radiation therapy is performed on the tongue.

### BRIEF DESCRIPTION OF DRAWINGS

Fig. 1 is a perspective view illustrating an upper side of a tongue suction restrainer according to a first embodiment.
Fig. 2 is a perspective view illustrating a lower side of the tongue suction restrainer according to the first embodiment.
Fig. 3 is a cross-sectional view illustrating a use state of the tongue suction restrainer according to the first embodiment.
Fig. 4 is a diagram illustrating a tongue suction restrainer manufacturing kit.
Fig. 5(A) is a cross-sectional view illustrating an upper fixing portion. Fig. 5(B) is a cross-sectional view illustrating a state in which a tongue suction portion is formed on a back surface side of a recess in the upper fixing portion. Fig. 5(C) is a cross-sectional view illustrating a tongue suction restrainer according to a second embodiment in which a lower fixing portion is formed on a lower jaw side of an upper fixing portion.
Fig. 6 is a perspective view illustrating a lower side of a tongue suction restrainer according to a third embodiment.
Fig. 7 is a cross-sectional view illustrating a use state of the tongue suction restrainer according to the third embodiment.
Fig. 8 is a cross-sectional view illustrating a use state of a tongue suction restrainer according to a fourth embodiment.

### DESCRIPTION OF EMBODIMENTS

Hereinafter, modes for carrying out the disclosure will be described with reference to the drawings. Configuration elements denoted by the same reference numerals in each drawing mean the same or similar configuration elements. Note that redundant descriptions and reference numerals in embodiments to be described below may be omitted. In addition, the drawings used in the following description are all schematic views, and a dimensional relationship of each element, a ratio of each element, the number of holes, and the like illustrated in the drawings do not necessarily coincide with actual dimensional relationship, ratio, number, and the like. In addition, a dimensional relationship of each element, a ratio of each element, and the like do not necessarily coincide among the plurality of drawings.

### [First Embodiment]

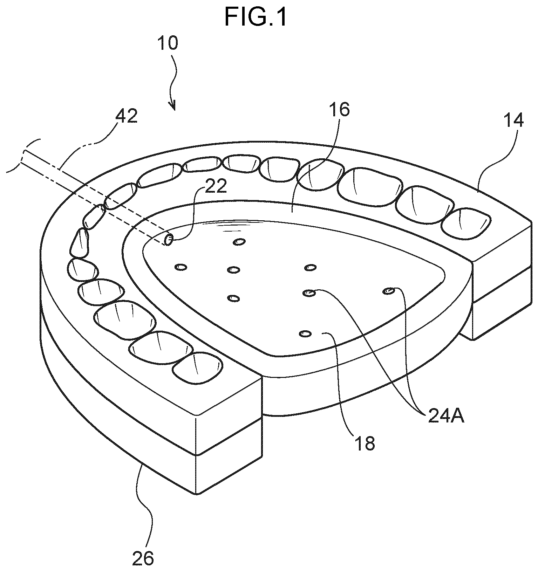

In Figs. 1 to 3, a tongue suction restrainer 10 according to the present embodiment is a spacer that is attached to an oral cavity 12 of a patient as an example of a user to suck and restrain a tongue 28, for example, at the time of MRI diagnosis of an inflammatory disease of the tongue 28 or stereotactic radiation therapy for a tongue cancer. The tongue suction restrainer 10 includes an upper fixing portion 14, an abutment portion 16, a recess 18, a suction portion 22, a tongue suction portion 24, and a lower fixing portion 26.

The upper fixing portion 14 is a member that is fixable to an upper jaw 32 of the patient when the tongue suction restrainer is attached to the oral cavity 12 of the patient. The upper fixing portion 14 may be fixable to the tooth 33 of the upper jaw 32 of the patient. In addition, the upper fixing portion 14 may be a so-called mouthpiece manufactured by molding the tooth 33 of the upper jaw 32 of each patient.

The abutment portion 16 is an annular portion that abuts on a hard palate 36 of the patient when the tongue suction restrainer is attached to the oral cavity 12 of the patient. The recess 18 is a portion that is formed at an inner side of the abutment portion 16 to form a closed space 38 with the hard palate 36 in a state in which the entire circumference of the abutment portion 16 abuts on the hard palate 36. Here, the abutment portion 16 abutting on the hard palate 36 means that the abutment portion 16 abuts on a portion including the hard palate 36, and does not prevent abutting on a portion other than the hard palate 36, for example, a soft palate.

The suction portion 22 is a portion that communicates with the recess 18 to apply a negative pressure to the closed space 38 from an outside. As an example, the suction portion 22 is a through-hole penetrating from a front surface corresponding to a space between upper and lower front teeth of the upper fixing portion 14 and the lower fixing portion 26 to the recess 18. For example, a catheter 42 for suction is connected to the suction portion 22, and thus, the negative pressure can be applied to the closed space 38 from the outside.

The tongue suction portion 24 is a portion that is formed at a position facing the tongue 28 of the patient when the tongue suction restrainer is attached to the oral cavity 12, has one or a plurality of suction holes 24A provided to communicate with the recess 18, and that sucks the tongue 28 by the negative pressure acting on the closed space 38. The tongue suction portion 24 is positioned, for example, on a back surface of the recess 18. In addition, the tongue suction portion 24 is, for example, a surface curved in a recessed shape in cross-sectional view in order to enhance restrainability of the tongue 28, but may have another shape.

The lower fixing portion 26 is a portion that is fixable to a tooth 35 of a lower jaw 34 of the patient, and that is fixable to, for example, the tooth 35 of the lower jaw 34 of the patient. The lower fixing portion 26 may be made of a compound that deforms in accordance with the tooth 35 of the lower jaw 34 when the upper and lower teeth 33 and 35 mesh with each other. In addition, similarly to the upper fixing portion 14, the lower fixing portion 26 may be manufactured by molding the tooth 35 of the lower jaw 34 of each patient.

The abutment portion 16, the recess 18, and the tongue suction portion 24 are made of, for example, a hard resin, and the upper fixing portion 14 is joined around the abutment portion 16. The lower fixing portion 26 is bonded to the lower jaw 34 side of the upper fixing portion 14.

### (Action)

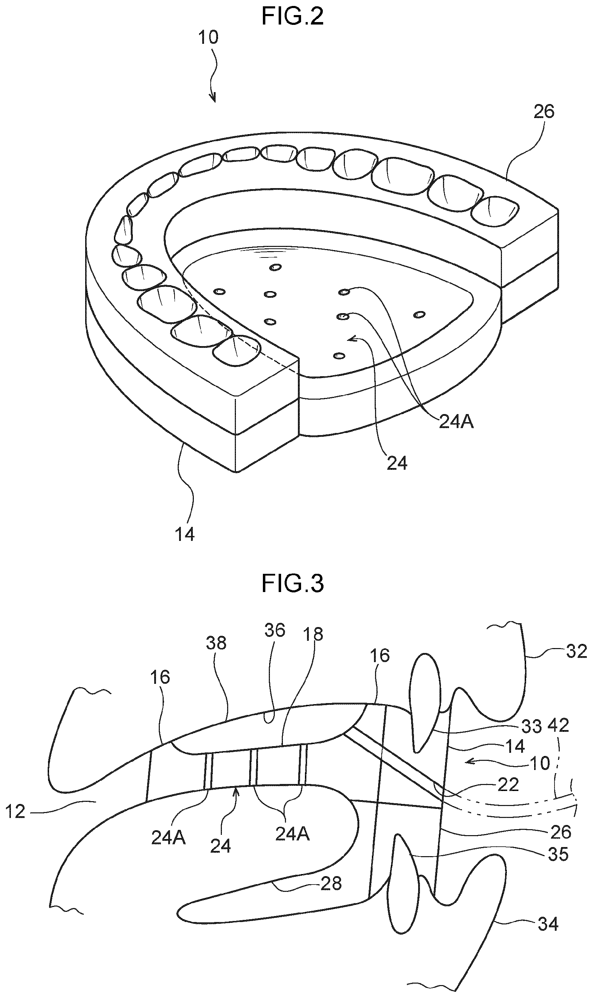

The present embodiment has the above configuration, and the action thereof will be described below. In Fig. 3, the tongue suction restrainer 10 according to the present embodiment is fixed to the upper jaw 32 of the patient by the upper fixing portion 14 when the tongue suction restrainer is attached to the oral cavity 12 of the patient, and the annular abutment portion 16 abuts on the hard palate 36 of the patient. Specifically, the upper fixing portion 14 is fixable to the tooth 33 of the upper jaw 32 of the patient. In addition, the lower fixing portion 26 is fixable to the tooth 35 of the lower jaw 34 of the patient. Thus, the tongue suction restrainer 10 can be accurately and stably attached to the oral cavity 12 of the patient.

In a state in which the entire circumference of the abutment portion 16 abuts on the hard palate 36, the closed space 38 is formed between the recess 18 at an inner side of the abutment portion 16 and the hard palate 36. The suction portion 22 communicates with the recess 18. For example, when the negative pressure is applied to the closed space 38 from the outside through the suction portion 22 by using the catheter 42, the abutment portion 16 is adsorbed to the hard palate 36. In addition, since this negative pressure acts on the tongue suction portion 24 through the suction hole 24A, a surface of the tongue 28 is sucked and restrained by the tongue suction portion 24. As a result, since the movement of the tongue 28 can be appropriately suppressed, irradiation accuracy of radiation when the stereotactic radiation therapy is performed on the tongue 28 can be improved. In addition, it is easy to observe the tongue 28 by, for example, magnetic resonance imaging (MRI).

### [Tongue suction restrainer manufacturing kit]

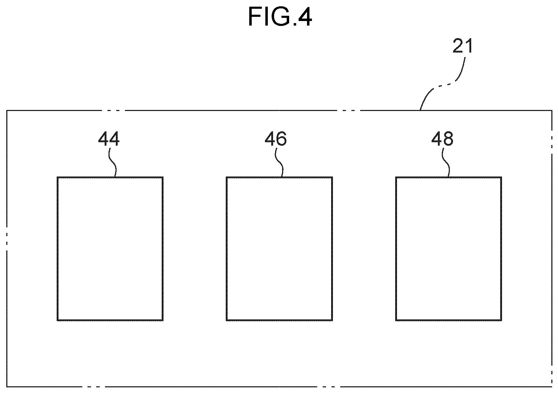

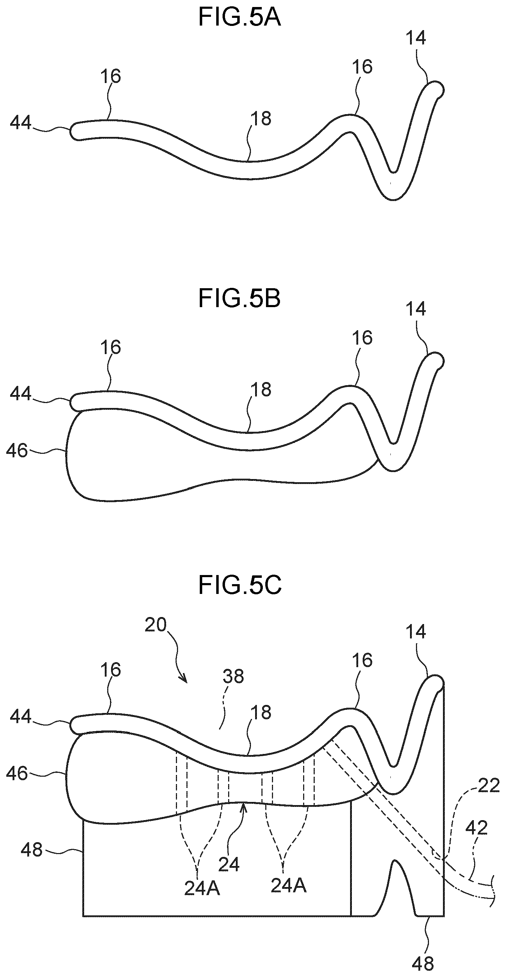

In Figs. 4 and 5, a tongue suction restrainer manufacturing kit 21 is a kit for manufacturing a tongue suction restrainer 20 (Fig. 5(C)) according to a second embodiment. Note that names and reference numerals of parts not illustrated in Figs. 4 and 5 correspond to the portions denoted by the same reference numerals in the first embodiment, and thus the description thereof will be omitted.

In Fig. 4, the tongue suction restrainer manufacturing kit 21 includes an upper fixing portion material 44, a tongue suction portion material 46, and a lower fixing portion material 48, which are packaged, for example.

The upper fixing portion material 44 is a material for forming the upper fixing portion 14, which covers and is fixed to the tooth 33 of the upper jaw 32 of the patient from a tooth mold of the upper jaw 32 of the patient. In addition, the upper fixing portion material 44 is a material for forming the annular abutment portion 16 abutting on the hard palate 36 of the patient and the recess 18 forming the closed space 38 between the abutment portion 16 and the hard palate 36 in a state in which the entire circumference of the abutment portion 16 abuts on the hard palate 36 at an inner side of the abutment portion.

The tongue suction portion material 46 is a material for forming the tongue suction portion 24, which is attached to the back surface side of the recess 18 and faces the tongue 28 of the patient.

The lower fixing portion material 48 is a material for forming the lower fixing portion 26, which is attached to the lower jaw 34 side of the upper fixing portion 14 and that covers and is fixed to the tooth 35 of the lower jaw 34 of the patient.

In the tongue suction restrainer manufacturing kit 21, the plurality of suction holes 24A communicating from the recess 18 to the tongue suction portion 24 and the suction portion 22 communicating with the recess 18 and applying the negative pressure to the closed space 38 from the outside can be formed.

Note that each material is, for example, a resin material such as a resin or a compound used for molding each part, but may be a resin component molded in advance. However, each material is not limited to these materials (resins), and includes, for example, wax and silicon.

### [Method of manufacturing tongue suction restrainer]

For example, the above tongue suction restrainer manufacturing kit 21 is used as a method of manufacturing the tongue suction restrainer 20. This manufacturing method includes
a step (Fig. 5(A)) of forming the upper fixing portion 14 that covers and is fixed to the tooth 33 of the upper jaw 32 of the patient from the tooth mold of the upper jaw 32 of the patient by the upper fixing portion material 44,
a step (Fig. 5(B)) of forming the tongue suction portion 24 facing the tongue 28 of the patient on the back surface side of the recess 18 in the upper fixing portion 14 by the tongue suction portion material 46,
a step (Fig. 5(C)) of forming the lower fixing portion 26 that covers and is fixed to the tooth 35 of the lower jaw 34 of the patient on the lower jaw 34 side of the upper fixing portion 14 by the lower fixing portion material 48, and
a step (Fig. 5(C)) of forming the suction hole 24A communicating from the recess 18 to the tongue suction portion 24 and the suction portion 22 communicating with the recess 18 and applying the negative pressure to the closed space 38 from the outside.

In the step of forming the upper fixing portion 14 from the tooth mold of the upper jaw 32, the tooth mold is taken by using a modeling material, and a tooth mold model is manufactured by using gypsum. At this time, it is suitable to form the recess 18 of Fig. 5(A) by performing dome-shaped build-up on a portion corresponding to the hard palate. When a sheet made of the upper fixing portion material 44 is heated by using a vacuum former, is adsorbed to the tooth mold model, and is cured, the upper fixing portion 14 having the recess 18 is obtained.

In the step of forming the tongue suction portion 24 on the back surface side of the recess 18 of the upper fixing portion 14, for example, a member having the tongue suction portion 24 formed by using the tongue suction portion material 46 is bonded to the back surface side of the recess 18 of the upper fixing portion 14. The tongue suction portion material 46 may be thickly applied to the back surface side of the recess 18 of the upper fixing portion 14 and is then cured.

In the step of forming the lower fixing portion 26 on the lower jaw 34 side of the upper fixing portion 14, for example, the lower fixing portion material 48 softened by heating is built on the lower jaw 34 side of the upper fixing portion 14, and the upper fixing portion 14 is attached to the upper jaw 32 (Fig. 3) of the patient and is lightly bitten. As a result, the tooth mold of the lower jaw 34 can be left in the lower fixing portion material 48. Note that a plurality of sizes of lower fixing portion materials 48 molded in advance according to the lower jaw 34 may be prepared and selected in accordance with the patient.

Then, in the final step, the plurality of suction holes 24A communicating with the tongue suction portion 24 from the recess 18 are formed, and the suction portion 22 communicating with the recess 18 and applying the negative pressure to the closed space 38 from the outside is formed.

In the tongue suction restrainer manufacturing kit 21, in Fig. 5(A), the upper fixing portion 14 is made of the upper fixing portion material 44. Subsequently, in Fig. 5(B), the tongue suction portion 24 is formed by the tongue suction portion material 46 on the back surface side of the recess 18 of the upper fixing portion 14. Subsequently, in Fig. 5(C), the lower fixing portion 26 is manufactured by the lower fixing portion material 48 on the lower jaw 34 side of the upper fixing portion 14. Then, the suction hole 24A penetrating from the recess 18 to the tongue suction portion 24 and the suction portion 22 are formed.

As described above, in this manufacturing method, the tongue suction restrainer corresponding to each patient can be easily manufactured by using the tongue suction restrainer manufacturing kit 21.

### [Third Embodiment]

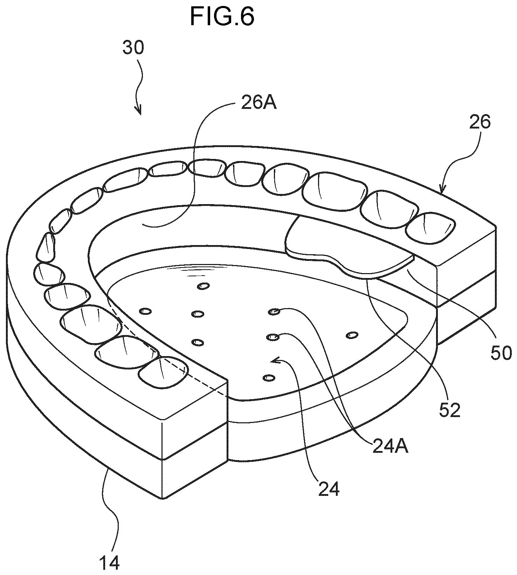

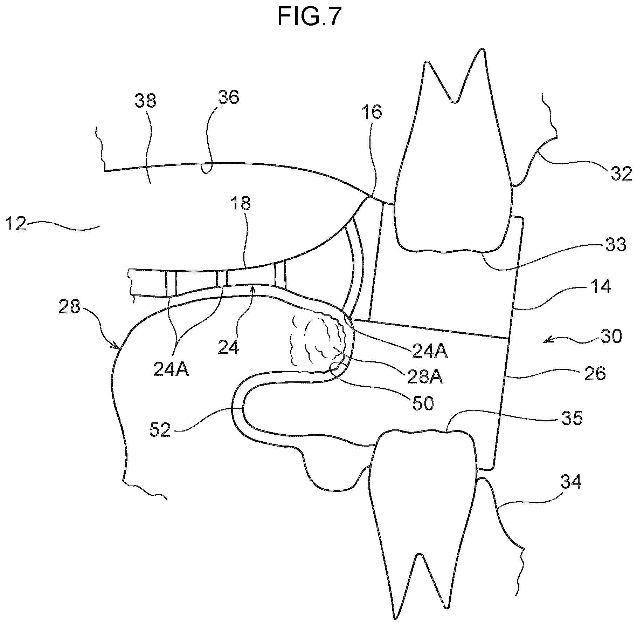

In Figs. 6 and 7, a tongue suction restrainer 30 according to the present embodiment has a protrusion 52 that protrudes from an inner peripheral portion 26A of the lower fixing portion 26 and that forms a pocket 50 for allowing a part of the tongue 28 to enter between the protrusion and the tongue suction portion 24.

For example, the protrusion 52 is formed in a shelf shape inward from a position of a substantially lower end of the inner peripheral portion 26A of the lower fixing portion 26. An end edge of the protrusion 52 is formed in, for example, a curved shape in consideration of an influence on the tongue 28. A position and a shape of the protrusion 52 are changed in accordance with a position and a size of a tumor 28A of the tongue 28, if appropriate.

The suction hole 24Ais also provided in a portion that forms the pocket 50 of the lower fixing portion 26. That is, the recess 18 and the pocket 50 communicate with each other through the suction hole 24A.

In the present embodiment, the pocket 50 is formed between the protrusion 52 provided on the inner peripheral portion 26A of the lower fixing portion 26 and the suction portion 22. A part of the tongue 28, for example, the tumor 28A is inserted into the pocket 50, and thus, the tumor 28A can be wrapped with the tongue suction restrainer. As a result, air artifacts on the lower jaw side can be reduced, and the tumor 28A can be easily observed by MRI.

In addition, since the suction hole 24A is also provided in the portion that forms the pocket 50 in the lower fixing portion 26, the tongue 28 can be sucked even in the pocket 50 to enhance adhesion between the tongue 28 and the tongue suction restrainer.

Since the other portions are similar to the portions of the first embodiment, the same portions are denoted by the same reference numerals in the drawings, and the description thereof will be omitted.

### [Fourth Embodiment]

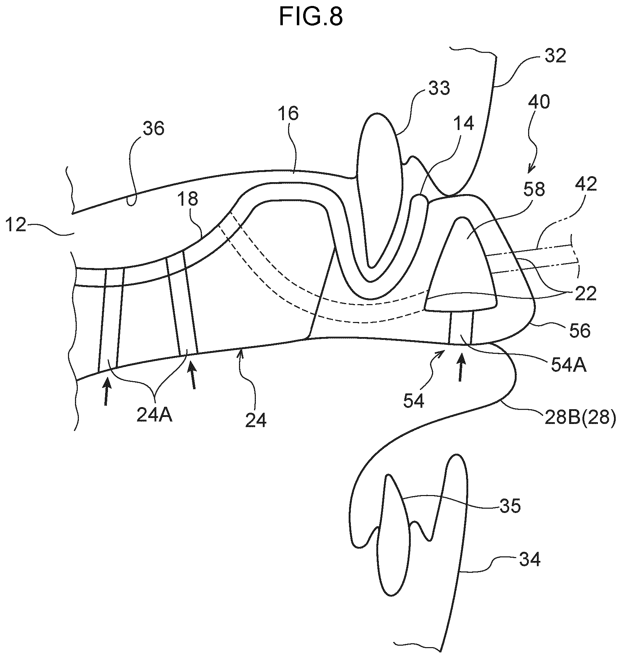

In Fig. 8, in the tongue suction restrainer 20 according to the present embodiment, a tongue suction portion 54 that sucks a distal end portion 28B of the tongue 28 and pulls out and restrains the distal end portion 28B to the outside of the oral cavity 12 is provided in addition to the tongue suction portion 24 that sucks the tongue 28 inside the oral cavity 12 when the tongue suction restrainer is attached. Specifically, a protruding portion 56 having a closed space 58 therein is provided further in front of a front edge of the upper fixing portion 14 (in front of the oral cavity 12 in an attached state for the patient), and a lower surface of the protruding portion 56 is the tongue suction portion 54. The suction portion 22 for applying the negative pressure from the outside communicates with the recess 18 (closed space 38) and the closed space 58. The tongue suction portion 54 and the closed space 58 communicate with each other through a suction hole 54A. The tongue suction portion 54 is positioned in front of the tooth 35 of the lower jaw 34 in the attached state. As a result, in a state in which the distal end portion 28B of the tongue 28 comes out of the oral cavity 12, the tumor formed at the distal end portion 28B can be irradiated with the radiation with high accuracy.

Since the other portions are similar to the portions of the first embodiment or the second embodiment, the same portions are denoted by the same reference numerals in the drawings, and the description thereof will be omitted.

According to the disclosure, it is possible to provide an improved tongue suction restrainer, a tongue suction restrainer manufacturing kit, and a method of manufacturing a tongue suction restrainer.

### [Other Embodiments]

Although the examples of the embodiments of the disclosure have been described above, the embodiments of the disclosure are not limited to the above embodiments, and it is a matter of course that various modifications can be made without departing from the gist of the disclosure in addition to the above embodiments. In addition, it is not hindered that the embodiments of the disclosure are used for other applications such as tongue fixation for applications other than the radiation therapy. For example, the tongue suction restrainers 10, 20, 30, and 40 can also be used for the radiation therapy by normal irradiation (general irradiation that does not apply to the definition of the stereotactic radiation therapy), intensity modulation radiation therapy, electron beam therapy, proton beam therapy, carbon beam therapy, or FLASH (registered trademark) irradiation which is a future therapy.

Although the upper fixing portion 14 is fixable to the tooth 33 of the upper jaw 32 of the patient, a target to be fixed need not be the tooth 33 as long as the upper fixing portion is fixable to the upper jaw 32.

The tongue suction restrainers 10, 20, and 30 have the lower fixing portion 26 that is fixable to the tooth 35 of the lower jaw 34 of the patient, but may have a configuration without the lower fixing portion as in a tongue suction restrainer 40 according to a fourth embodiment. In addition, in the fourth embodiment, the lower fixing portion 26 that is fixable to the tooth 35 of the lower jaw 34 may be provided.

Note that the method of manufacturing the tongue suction restrainer is not limited to the above-described manufacturing method, and may be a manufacturing method using a shaping method such as a 3D printer, a resin molding technique, a cutting processing technique, or the like.

The disclosure of Japanese Patent Application No. 2022-158216 filed on September 30, 2022 is incorporated herein by reference in its entirety.

All documents, patent applications, and technical standards described in this specification are incorporated herein by reference to the same extent as if each individual document, patent application, and technical standard were specifically and individually indicated to be incorporated by reference.

## Claims

1. A tongue suction restrainer, comprising:
an upper fixing portion that is fixable to an upper jaw of a user when the tongue suction restrainer is attached to an oral cavity of the user;
an annular abutment portion that abuts a hard palate of the user when the tongue suction restrainer is attached to the oral cavity;
a recess that is formed at an inner side of the abutment portion to form a closed space with the hard palate in a state in which an entire circumference of the abutment portion abuts the hard palate;
a suction portion that communicates with the recess to apply a negative pressure to the closed space from an outside; and
a tongue suction portion that is formed at a position facing a tongue of the user when the tongue suction restrainer is attached to the oral cavity, that has a suction hole provided to communicate with the recess, and that sucks the tongue by the negative pressure.

2. The tongue suction restrainer according to claim 1, wherein the upper fixing portion is fixable to a tooth of the upper jaw of the user.

3. The tongue suction restrainer according to claim 1, further comprising a lower fixing portion that is fixable to a tooth of a lower jaw of the user.

4. The tongue suction restrainer according to claim 3, further comprising:
a protrusion that protrudes from an inner peripheral portion of the lower fixing portion to form a pocket for allowing a part of the tongue to enter between the protrusion and the tongue suction portion,
wherein the suction hole is provided in a portion of the lower fixing portion which forms the pocket.

5. A tongue suction restrainer manufacturing kit, comprising:
an upper fixing portion material for forming an upper fixing portion that covers and is fixed to a tooth of an upper jaw of a user from a tooth mold of the upper jaw of the user, and forming an annular abutment portion that abuts a hard palate of the user, and a recess that forms a closed space with the hard palate at an inner side of the abutment portion in a state in which an entire circumference of the abutment portion abuts the hard palate;
a tongue suction portion material that is attached to a back surface side of the recess and that forms a tongue suction portion facing a tongue of the user; and
a lower fixing portion material for forming a lower fixing portion that is attached to a lower jaw side of the upper fixing portion and that covers and is fixed to a tooth of a lower jaw of the user,
wherein a suction hole communicating from the recess to the tongue suction portion and a suction portion communicating with the recess to apply a negative pressure to the closed space from an outside are formed.

6. A method of manufacturing a tongue suction restrainer by using the tongue suction restrainer manufacturing kit according to claim 5, comprising:
a step of forming, by the upper fixing portion material, an upper fixing portion that covers and is fixed to a tooth of an upper jaw of the user from a tooth mold of the upper jaw of the user;
a step of forming, by the tongue suction portion material, a tongue suction portion facing a tongue of the user on a back surface side of the recess in the upper side fixing portion;
a step of forming, by the lower fixing portion material, a lower fixing portion that covers and is fixed to a tooth of the lower jaw of the user on a lower jaw side of the upper fixing portion; and
a step of forming a suction hole communicating with the tongue suction portion from the recess, and a suction portion communicating with the recess and applying a negative pressure to the closed space from an outside.
